# EUROPÄISCHE PATENTSCHRIFT

(11) **EP 2 442 777 B1**
(45) Veröffentlichungstag und Bekanntmachung des Hinweises auf die Patenterteilung: **27.03.2019**
(21) Anmeldenummer: 10784430.0
(22) Anmeldetag: 15.06.2010
(51) Int. Cl.: A61K 6/00

(54) **VERFAHREN ZUR HERSTELLUNG EINES HAFTMITTELS FÜR ZAHNPROTHESEN**
PROCESS OF MANUFACTURE OF AN ADHESIVE COMPOSITION FOR DENTAL PROSTHESES
PROCÉDÉ DE FABRICATION D'UNE COMPOSITION ADHESIVE POUR PROTHÈSES DENTAIRES

(30) Priorität: 18.06.2009 AT 8602010
(43) Veröffentlichungstag der Anmeldung: 25.04.2012
(73) Patentinhaber: Altwirth, Ella, 2340 Mödling (AT)
(72) Erfinder: Altwirth, Ella, 2340 Mödling (AT); Altwirt, Oskar, Verstorben (AT)
(74) Vertreter: Patentanwaltskanzlei Hübscher
(86) Internationale Anmeldenummer: PCT/AT2010/000217
(87) Internationale Veröffentlichungsnummer: WO 2010/144934

(56) Entgegenhaltungen:
- EP-A2- 0 995 420
- DE-A1- 3 546 367
- US-A- 4 318 742
- US-A- 5 001 170
- US-A1- 2007 185 237

## Beschreibung

Die Erfindung bezieht sich auf ein Haftmittel für Zahnprothesen mit einer pastösen, gummiartigen Trägersubstanz, die ausschließlich aus Lebensmittelzutaten besteht.

Aus der US 4 318 742 ist ein Haftmittel für Zahnprothesen mit einer aus Festharzen bestehenden Trägersubstanz bekannt, die Polyvinylacetat, ein Glycerinester aus Wurzelharz und Carboxymethylcellulose enthält. Eine aus der DE 35 46 367 bekannte Zusammensetzung enthält Alginat, Polyvinylacetat und Carboxymethylcellulose und eine aus der EP 0 995 420 bekannte Zusammensetzung enthält ein natürliches Glycerinresinat und ein Hydrokolloid aus beispielsweise Carboxymethylcellulose. Eine weitere Zusammensetzung aus der US 5 001 170 enthält eine Mischung aus künstlichen, wasserunlöslichen Polymeren, Hydroxypropylcellulose und weiteren Substanzen.

Die Trägersubstanz der vorliegenden Erfindung besteht aus zwei verschiedenen Festharzen, die miteinander vermischt eine gummiartige Substanz entstehen lassen, die einen festen Halt der Zahnprothese gewährleisten. Als Lösungsmittel wird unvergällter Alkohol, sowie Kokosfett verwendet.

Zur Anwendung kommt auch eine Carboxymethylcellulose, eine Cellulose, die unter anderem für Speiseeis verwendet wird.

Um eine beruhigende Wirkung auf die Gingiva (Zahnfleisch) zu erreichen, ist dem Haftmittel ein Kamillenblütenextrakt hinzugefügt, sowie ein Vitamin C (Ascorbinsäure) in gut abgestimmter Dosis.

Bei den Festharzen handelt es sich erstens um ein Polyvinylacetat, das in der Kaugummiherstellung verwendet wird und als Lebensmittel eingestuft ist, und unter dem Markennamen Vinnapas B60 Spezial bekannt ist.

Vinnapas B60 Spezial ist ein festes, thermoplastisches, hochmolekulares Homopolymer. Das klare und farblose Kunstharz wird durch Polymerisation von Vinylacetat hergestellt. Vinnapas B60 Spezial ist frei von Geschmack.

Vinnapas B60 Spezial entspricht der Zusatzstoff-Zulassungsverordnung (ZzuIV) FDA 21 CFR § 172.6105, sowie den Anforderungen in der Spezifikation für Polyvinylacetat, wie im Food Chemicals Codex (FCC), 5. Ausgabe 2004, festgelegt. Vinnapas B60 Spezial ist koscher zertifiziert.

Weiters ist auch ein Polyvinylacetat i.S. der VO (EG) Nr. 1782-002 mit Vorbehalt verwendbar.

Als zweites Festharz kommt ein Glycerinester aus Wurzelharz zur Anwendung, das auch als "Ester of Rosin" oder unter der Markenbezeichnung Ref.: VM/919 VALROSIN D bekannt ist, das in der Getränkeindustrie (Limonadenherstellung) und zur Herstellung von Kaugummi Verwendung findet und ebenfalls als Lebensmittel eingestuft ist.

Hinsichtlich des Stands der Technik ist aus dem Patent AT 407.827 B (Erfinder: Oskar Altwirth) ein Haftmittel für Zahnprothesen bekannt, das physiologisch unbedenklich ist. Es hat sich jedoch herausgestellt, dass dieses Haftmittel keine ausreichende Haftwirkung aufweist und aufwändig zu reinigen ist.

Der Erfindung liegt somit die Aufgabe zugrunde, ein Haftmittel der eingangs geschilderten Art so zu gestalten, dass eine optimale Haftwirkung gewährleistet wird, und gleichzeitig eine leichte Reinigung ermöglicht wird.

Die Erfindung löst diese Aufgabe über ein neues Herstellungsverfahren gemäß Patentanspruch 1, das über eine Verbindung beider Harze einen inneren Zusammenhalt herstellt, sodass die Trägersubstanz nicht so leicht durch die Speicheleinwirkung aufgelöst werden kann. Ein Festharz alleine (Valrosin) kann diese Aufgabe nicht lösen, wie es sich in der Anmeldung WO 2005/044202 Erfinder Oskar Altwirth, gezeigt hat.

Die Herstellung einer bestmöglichen Trägersubstanz geschieht auf folgende Weise durch eine Abfolge von Mischvorgängen:

### 1. Mischvorgang :

100% Polyvinylacetat werden mit 50% Alkohol (96 %ig) bei einer Temperatur von 80°C vermischt, bis eine flüssige Lösung entsteht.

### (Beispiel: 100 Gramm Polyvinylacetat werden mit 50 Gramm Alkohol bei einer Temperatur von 80°C vermischt)

### 2. Mischvorgang:

100% Ester of Rosin (Valrosin) werden mit 80% Kokosfett bei einer Temperatur von 100°C aufgelöst bzw. verflüssigt, sodass nach dem Erkalten dieser Mischung eine pastöse Masse entsteht. Das Erkalten bei Zimmertemperatur kann bis zu 8 Stunden dauern.

(Beispiel: 100 Gramm Ester of Rosin werden mit 80 Gramm Kokosfett vermischt, auf 100°C erhitzt, bis sich beide Substanzen verflüssigt haben, anschließend muss die Masse erkalten, bis eine pastöse Masse entsteht)

### 3. Mischvorgang:

In 100% Alkohol (96 %ig) werden 2% Hydroxypropylcellulose (Klucel) eingerührt. Diese zweite Mischung benötigt eine Standzeit von ca. 3 Tagen, bis eine klare Masse mit einer gallertartigen Konsistenz entstanden ist.

### (Beispiel: In 100 Gramm Alkohol (96 %ig) werden 2 Gramm Hydroxypropylcellulose eingerührt)

### 4. Mischvorgang:

In 100% der ersten erkalteten Mischung des ersten Mischvorganges werden 20% der zweiten Mischung des zweiten Mischvorganges eingerührt.

### 5. Mischvorgang:

In 100% der ersten und zweiten Mischung werden 70% Carboxymethylcellulose (CMC) eingerührt. Für diesen Mischvorgang wird ein geschlossenes Planetenrührwerk benötigt, damit ein Verlust des Alkohols und der CMC vermieden wird, und um eine gleichbleibende Qualität gewährleisten zu können. Die Mischdauer hängt von der Quantität des Mischverhältnisses ab.

### 6. Mischvorgang:

Der gesamten Masse des fünften Mischvorganges werden abschließend 4% Hydroxypropylcellulose (Klucel) beigemengt (Beispiel: 100 Gramm Festharzmischung werden 4 Gramm Hydroxypropylcellulose beigemengt). Das Einbringen der Hydroxypropylcellulose ist äußerst kompliziert, da eine Verklumpung der Masse entstehen kann, und daher beim Rührvorgang die Masse wieder auf 60°C erwärmt werden muss, und zwar bei einem vollkommen geschlossenen Rührwerk.

Beide Festharze sind nicht wasserlöslich, wie z.B. bei Speichel oder Getränken. Das hat unter Anderem höchste Priorität für den Halt der Zahnprothese. Um jedoch eine Reinigung zu ermöglichen, wird in diesem letzten Mischvorgang Hydroxypropylcellulose (Klucel) beigemengt.

Das erfindungsgemäß erzeugte Zahnprothesen-Haftmittel besteht aus den als Lebensmittel deklarierten zwei Grundmassen Polyvinylacetat und Ester of Rosin, und zeichnet sich aufgrund der Wasserunlöslichkeit des Haftmittels durch eine hohe Haftfähigkeit aus.

Durch die Hinzugabe von Hydroxypropylcellulose wird das Haftmittel nicht nur geschmeidiger, sondern die Reinigung der Zahnprothesen wird erheblich vereinfacht. Vorteilhaft ist des Weiteren die Beimengung von Kieselsäure (Silica). Ein Prothesenträger ist sicherlich bereit ein natürliches Haftmittel zu kaufen, das über eine ausreichende Haftkraft verfügt, die den chemischen Haftmitteln überlegen ist, und außerdem aufgrund der gesundheits-unbedenklichen Festharze dem gesundheitlichen Wohl des Protheseträgers dient.

## Patentansprüche

1. Verfahren zur Herstellung eines Haftmittels für Zahnprothesen, **dadurch gekennzeichnet, dass**
-) in einem ersten Verfahrensschritt 10 Gewichtsteile Polyvinylacetat mit 5 Gewichtsteilen 96 %igem Alkohol bei einer Temperatur von 80°C vermischt werden, bis eine flüssige Lösung entsteht,
-) in einem zweiten Verfahrensschritt 10 Gewichtsteile Glycerinester aus Wurzelharz mit 8 Gewichtsteilen Kokosfett bei einer Temperatur von 100°Caufgelöst bzw. verflüssigt werden, sodass nach dem Erkalten dieser Mischung eine pastöse Masse entsteht,
-) in einem dritten Verfahrensschritt in 10 Gewichtsteilen 96 %igem Alkohol 0,2 Gewichtsteile Hydroxypropylcellulose eingerührt werden,
-) in einem vierten Verfahrensschritt in 10 Gewichtsteilen der erkalteten Mischung des ersten Verfahrensschrittes 2 Gewichtsteile der erkalteten Mischung des zweiten Verfahrensschrittes eingerührt werden,
-) in 10 Gewichtsteile der gesamten Masse des vierten Verfahrensschrittes 7 Gewichtsteile Carboxymethylcellulose (CMC) eingerührt werden,
-) und in einem abschließenden Verfahrensschritt die gesamte Masse des 5. Verfahrensschrittes auf 60°C erwärmt wird und je 10 Gewichtsteilen der gesamten Masse des 5. Verfahrensschrittes 0,4 Gewichtsteile Hydroxypropylcellulose beigemengt werden.

2. Gemäß einem Verfahren nach Anspruch 1 hergestelltes Haftmittel für Zahnprothesen mit einer aus Festharzen bestehenden Trägersubstanz umfassend Polyvinylacetat, ein Glycerinester aus Wurzelharz, sowie Carboxymethylcellulose und Hydroxypropylcellulose.

3. Haftmittel nach Anspruch 2, **dadurch gekennzeichnet, dass** es zusätzlich Kieselsäure enthält.

## Claims

1. Method for producing an adhesive for dental prostheses, **characterised in that**
-) in a first method step, 10 parts by weight of polyvinyl acetate are mixed with 5 parts by weight of 96% alcohol at a temperature of 80°C until a liquid solution is produced,
-) in a second method step, 10 parts by weight of glycerol ester of wood rosin are dissolved or liquefied with 8 parts by weight of coconut fat at a temperature of 100°C such that after cooling of this mixture a paste-like mass is produced,
-) in a third method step, 0.2 parts by weight of hydroxypropyl cellulose are stirred into 10 parts by weight of 96% alcohol,
-) in a fourth method step, 2 parts by weight of the cooled mixture from the second method step are stirred into 10 parts by weight of the cooled mixture from the first method step,
-) 7 parts by weight of carboxymethyl cellulose (CMC) are stirred into 10 parts by weight of the entire mass from the fourth method step,
-) and in a final method step, the entire mass from the 5^{th} method step is heated to 60°C and in each case 0.4 parts by weight of hydroxylpropyl cellulose are added to 10 parts by weight of the entire mass from the 5^{th} method step.

2. Adhesive for dental prostheses which is produced according to a method as claimed in claim 1 and comprises a carrier substance consisting of solid resins and comprising polyvinyl acetate, a glycerol ester of wood rosin, and carboxymethyl cellulose and hydroxypropyl cellulose.

3. Adhesive as claimed in claim 2, **characterised in that** it additionally contains silicic acid.

## Revendications

1. Procédé pour la fabrication d'un adhésif pour prothèses dentaires, **caractérisé en ce que** :
- dans une première étape de procédé, 10 parties en poids de polyvinylacétate sont mélangées avec 5 parties en poids d'alcool à 96 %, à une température de 80 °C, jusqu'à l'obtention d'une solution liquide,
- dans une deuxième étape de procédé, 10 parties en poids d'ester glycérique à base de colophane sont dissoutes ou liquéfiées avec 8 parties en poids de graisse de coco à une température de 100 °C de façon à obtenir une masse pâteuse après refroidissement de ce mélange,
- dans une troisième étape de procédé, 0,2 partie en poids d'hydroxypropylcellulose est délayée dans 10 parties en poids d'alcool à 96 %,
- dans une quatrième étape de procédé, 2 parties en poids du mélange refroidi de la deuxième étape de procédé sont délayées dans 10 parties en poids du mélange refroidi de la première étape de procédé,
- 7 parties en poids de carboxyméthylcellulose (CMC) sont délayées dans 10 parties en poids de la masse totale de la quatrième étape de procédé,
- et dans une étape de procédé ultérieure, la masse totale de la 5^{ème} étape de procédé est chauffée à 60°C et chaque 10 parties en poids de la masse totale de la 5^{ème} étape de procédé sont ajoutées à 0,4 partie en poids d'hydroxypropylcellulose.

2. Adhésif pour prothèses dentaires, fabriqué selon un procédé selon la revendication 1, comportant une substance support composée de résines solides, comprenant du polyvinylacétate, un ester glycérique à base de colophane, ainsi que du carboxyméthylcellulose et de l'hydroxypropylcellulose.

3. Adhésif selon la revendication 2, **caractérisé en ce qu'**il comprend, en outre, de la silice.
